# EUROPÄISCHE PATENTSCHRIFT

(11) **EP 0 640 088 B1**
(45) Veröffentlichungstag und Bekanntmachung des Hinweises auf die Patenterteilung: **09.10.1996**
(21) Anmeldenummer: 93909921.4
(22) Anmeldetag: 07.05.1993
(51) Int. Cl.: C07D 513/04, A61K 31/505

(54) **NEUE TRICYCLISCHE THIAZOLO- UND THIAZINO-DERIVATE UND DIESE ENTHALTENDE ANITVIRALE ARZNEIMITTEL**
NOVEL TRICYCLIC THIAZOLO AND THIAZINO DERIVATIVES AND ANTI-VIRAL MEDICAMENTS CONTAINING THEM
NOUVEAUX DERIVES THIAZOLIQUES ET THIAZINIQUES TRICYCLIQUES, ET MEDICAMENTS ANTIVIRAUX CONTENANT CES DERIVES

(30) Priorität: 10.05.1992 DE 4214830
(43) Veröffentlichungstag der Anmeldung: 01.03.1995
(73) Patentinhaber: BOEHRINGER MANNHEIM GMBH, 68298 Mannheim (DE)
(72) Erfinder: MERTENS, Alfred, D-6905 Schriesheim (DE); ZILCH, Harald, D-6800 Mannheim 31 (DE); KÖNIG, Bernhard, D-8137 Berg3 (DE); LESER, Ulrike, D-8000 München 70 (DE)
(86) Internationale Anmeldenummer: EP9301125
(87) Internationale Veröffentlichungsnummer: WO9323407

(56) Entgegenhaltungen:
- EP-A- 0 530 994
- WO-A-92/13863
- US-A- 3 891 638

## Beschreibung

Gegenstand der vorliegenden Erfindung sind tricyclische Thiazolo- und Thiazino-Derivate, Verfahren zu deren Herstellung und Arzneimittel, die diese Verbindungen enthalten.

Die Erfindung betrifft tricyclische Thiazolo[3,2-c]pyrimidin - und Thiazino[3,2-c]pyrimidin-Derivate der Formel I 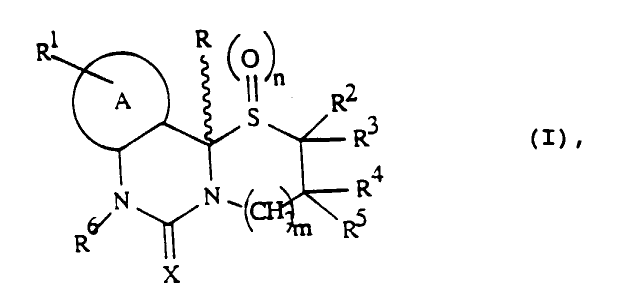 in der
- A: einen carbocyclischen Ring mit 5 oder 6 Kohlenstoffatomen oder einen heterocyclischen Ring mit max. 4 Heteroatomen darstellt, wobei die Heteroatome gleich oder verschieden sein können und Sauerstoff, Stickstoff oder Schwefel bedeuten, und die Heterocyclen gegebenenfalls an einem oder mehreren Stickstoffatomen ein Sauerstoffatom tragen können, und A durch einen oder mehrere Reste R¹, die gleich oder verschieden sein können, gegebenenfalls substituiert ist,
- x: ein Sauerstoff- oder Schwefelatom oder die Gruppe =NH, =N-C₁-C₆-Alkyl oder =S(O)₂ sein kann,
- R: einen geradkettigen oder verzweigten, gesättigten oder ungesättigten aliphatischen Rest mit 1-9 C-Atomen, der durch Phenyl substituiert sein kann, oder
einen Phenylring bedeutet,
oder einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, und die vorgenannten Phenylringe, die mono-, bi- oder tricyclischen carbocyclischen Ringe oder das heterocyclische mono-, bi- oder tricyclische Ringsystem gegebenenfalls ein- oder mehrfach substituiert ist durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylamino-carbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl,
- R¹: ein Wasserstoffatom, einen geradkettigen oder verzweigten, gesättigten oder ungesättigten aliphatischen Rest mit 1-6 C-Atomen oder C₁-C₆-Alkoxy, C₁-C₆-Alkylmercapto, C₁-C₆-Alkylsulfinyl, C₁-C₆-Alkylsulfonyl, Amino, C₁-C₆-Alkylamino, Di-C₁-C₆-Alkylamino, Sulfonamido, C₁-C₆-Alkoxycarbonyl, Carboxy, Halogen, Hydroxy, Nitro, Cyano, Azido, Phenyl oder Benzyloxy bedeutet,
- R², R³, R⁴ und R⁵: unabhängig voneinander gleich oder verschieden sein können und jeweils Wasserstoff, C₁-C₆-Alkyl, C₁-C₆-Hydroxyalkyl, Cyano, Carboxy oder C₁-C₆-Alkoxycarbonyl oder R² und R⁴ eine weitere Bindung zwischen den C-Atomen, an die sie gebunden sind, bedeuten können,
- R⁶: Wasserstoff oder C₁-C₆-Alkyl bedeutet,
- n: 0, 1 oder 2 bedeutet und
- m: 0 oder 1 bedeutet,
sowie deren Tautomere, Enantiomere, Diastereomere und physiologisch verträgliche Salze.

Verbindungen ähnlicher Struktur (Chinazolin-Derivate) sind aus der früheren Europäischen Patentanmeldung EP 0,530,994 als Inhibitoren der reversen Transkriptase bekannt. In den dort beschriebenen Beispielen 5 C - 5 F und 101 werden insbesondere die der Formel I entsprechenden Oxazolo[3,2-c]pyrimidine beschrieben, wobei A einen substituierten Phenylring darstellt und R einen Cylcopentyl-, Cyclopropyl- oder Phenylring oder eine Propylgruppe darstellt. Gegenüber diesen Verbindungen zeigen die erfindungsgemäßen Thiazolo[3,2-c]pyrimidine (m=0) und Thizino[3,2-c]pyrimidine (m=1) eine erhöhte pharmakologische Wirksamkeit.

Der vorliegenden Erfindung lag die Aufgabe zugrunde, neue tricylische Thiazolo[3,2-c]pyrimidine und Thiazino[3,2-c]pyrimidine zur Verfügung zu stellen, die insbesondere als pharmazeutische Wirkstoffe zur Herstellung von Arzneimitteln eingesetzt werden können.

Die Verbindungen der vorliegenden Erfindung weisen wertvolle pharmakologische Eigenschaften auf. Insbesondere besitzen sie eine antivirale Wirkung und eignen sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Die erfindungsgemäßen Verbindungen der allgemeinen Formel I besitzen eine ausgeprägte antivirale Wirkung und eignen sich insbesondere zur Behandlung von viralen bzw. retroviralen Infektionen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Es konnte nun nachgewiesen werden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutica besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

Die Verbindungen der Formel I können als Racemate oder als optisch aktive Derivate vorliegen.

Die Trennung der Racemate in die Enantiomeren kann analytisch, semipräparativ und präparativ chromatographisch auf geeigneten optisch aktiven Phasen mit gängigen Elutionsmitteln durchgeführt werden.

Als optisch aktive Phasen eignen sich beispielsweise optisch aktive Polyacrylamide oder Polymethacrylamide, z.T. auch an Kieselgel (z.B. ChiraSpher^{R} von Merck, Chiralpak^{R} OT/OP von Baker), Celluloseester/-carbamate (z.B. Chiracel^{R} OB/OY von Baker/Daicel), Phasen auf Cyclodextrin- oder Kronenetherbasis (z.B. Crownpak^{R} von Daicel) oder mikrokristallines Cellulosetriacetat (Merck).

In der Definition von A bedeutet A einen carbocyclischen Ring, insbesondere einen Phenyl- oder Cyclohexyl- oder Cyclopentylring. Der annelierte aromatische heterocyclische Ring A besitzt 5-6 Kohlenstoffatome, wobei bis zu 4 dieser Ringatome durch die Heteroatome Sauerstoff, Schwefel und/oder Stickstoff ersetzt sein können. Beispielhaft seien die folgenden Heterocyclen genannt: der Furan-, Thiophen-, Pyrrol-, Oxazol-, Isoxazol-, Thiazol-, Pyrazol-, Imidazol-, Oxadiazol-, Triazol-, Pyridin-, Pyridazin-, Pyrimidin- oder Pyrazinring. Sofern im heterocylischen Ring A ein Stickstoffatom vorhanden ist, können die entsprechenden Heterocyclen auch in Form ihrer N-Oxide vorliegen. Der Ring A kann durch einen oder mehrere, vorzugsweise ein, zwei oder drei Reste R¹ substitiert sein, wobei die Substituenten R¹ gleich oder verschieden sein können.

Ein aliphatischer Rest R oder R¹ bedeutet einen geradkettigen oder verzweigten Alkyl-, Alkenyl- oder Alkinylrest mit 1-9, vorzugsweise 2-7 Kohlenstoffatomen, wie z.B. der Propyl-, Isopropyl-, Butyl-, Isobutyl-, Pentyl-, Hexyl- oder Heptylrest. Als ungesättigte Reste kommen C₂-C₇-Alkenyl- und Alkinylreste in Frage, bevorzugt C₂-C₅, wie z.B. der Allyl-, Dimethylallyl-, Butenyl-, Isobutenyl-, Pentenyl- oder Propinylrest.

Ein aliphatischer Rest R, der durch Phenyl substituiert sein kann, ist insbesondere eine Phenyl-C₁-C₆-alkylgruppe, wie z.B. der Benzyl-, Phenethyl-, Phenylpropyl- oder Phenylbutylrest.

Enthalten die Reste R oder R¹ einen Phenylring, so kann dieser ein-, zwei- oder dreifach substituiert sein. Die Substituenten können unabhängig voneinander in o-, m- oder p-Stellung stehen.

Ein carbocyclischer Ring R mit 7-15 C-Atomen kann mono-, bi- oder tricyclisch sein und pro Ring jeweils 5 oder 6 C-Atome aufweisen. Dieser Ring kann gesättigt, ungesättigt, teilweise gesättigt oder aromatisch sein. Beispielhaft genannt seien die folgenden Ringsysteme: der Naphthyl-, Anthracenyl-, Phenanthrenyl-, Flourenyl-, Indenyl-, Indanyl-, Acenaphthylenyl-, Norbornyl-, Adamantylring oder eine C₃-C₇-Cycloalkyl- oder C₅-C₈-Cycloalkenylqruppe. Der carbocyclische Ring kann darüberhinaus mono- oder disubstituiert sein, wobei im Falle der Phenylringe die Substituenten unabhängig voneinander bevorzugt in o- oder m-Stellung stehen können.

Die heterocylischen mono-, bi- oder tricyclischen Ringsysteme des Restes R enthalten pro Ring 5 oder 6 Kohlenstoffatome, wobei 1-4 bzw. 1-5 C-Atome durch die Heteroatome Sauerstoff, Schwefel und/oder Stickstoff ersetzt sein können. Die Ringsysteme können aromatisch, partiell oder vollständig hydriert sein. Beispielhaft genannt seien die folgenden Ringsysteme: das Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei die ungesättigten bzw. aromatischen Carbo- und Heterocyclen partiell oder vollständig hydriert sein können. Sofern diese heterocyclischen Ringe ein Stickstoffatom enthalten, können die entsprechenden Heterocyclen auch in Form ihrer N-Oxide vorliegen. Das heterocyclische Ringsystem kann darüberhinaus mono- oder disubstituiert sein, wobei die Substituenten unabhängig voneinander bevorzugt in o- oder m-Stellung stehen können.

R bedeutet bevorzugt unsubstituiertes Phenyl oder Phenyl ein- oder zweifach substituiert durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁-C₆ Alkylaminocarbonyl, C₁-C₆-Alkoxycarbonyl-, Amino, Hydroxy, Nitro, Azido, Trifluormethyl, Cyano oder Halogen, wobei die zuvor genannten aliphatischen Reste bevorzugt bis zu 3 Kohlenstoffatomen enthalten.

Carbocyclische Ringe R sind bevorzugt Biphenyl, Naphthyl, Anthracenyl, Indenyl, Fluorenyl, Acenaphthylenyl, Phenanthrenyl, Norbornyl, Adamantyl, C₃-C₆-Cycloalkyl, C₅-C₈-Cycloalkenyl, wobei die carbocyclischen Ringe ein- oder zweifach substituiert sein können durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen, wobei die zuvor genannten aliphatischen Reste bevorzugt bis zu 3 Kohlenstoffatomen enthalten.

Heterocyclische Ringsysteme R sind bevorzugt Pyrrol, Imidazol, Furan, Thiophen, Pyridin, Pyrimidin, Thiazol, Triazin, Indol, Chinolin, Isochinolin, Cumaron, Thionaphthen, Benzimidazol, Chinazolin, Methylendioxybenzol, Ethylendioxybenzol, Carbazol, Acridin und Phenothiazin, wobei die heterocyclischen Ringe ein- oder zweifach substituiert sein können durch C₁-C₃-Alkyl, C₁-C₃-Alkoxy, C₁-C₃-Alkylmercapto, C₁-C₃-Alkylsulfinyl, C₁-C₃-Alkylsulfonyl, C₂-C₄-Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

Für den Rest R¹ ist Wasserstoff, C₁-C₃-Alkyl, C₂-C₄-Alkenyl, C₂-C₄-Alkinyl, C₁-C₃-Alkoxy, C₁-C₃-Alkylmercapto, C₁-C₃-Alkylamino, C₁-C₃-Alkoxycarbonyl, Amino, Halogen, Hydroxy, Nitro, Cyano und Azido bevorzugt.

Bevorzugte Substituenten für R², R³, R⁴ und R⁵ sind Wasserstoff, C₁-C₃-Alkyl, C₁-C₃-Hydroxyalkyl, Carboxy, C₁-C₃-Alkoxycarbonyl und Cyano oder wenn R² und R⁴ eine zusätzliche Bindung bilden.

X ist bevorzugt Sauerstoff. Unter Halogen ist allgemein Fluor, Chlor, Brom und Iod zu verstehen, bevorzugt Fluor, Chlor und Brom.

Bevorzugte annelierte Heterocyclen A sind aromatische, stickstoffhaltige Ringe mit 5 oder 6 Ringatomen.

Besonders bevorzugte Reste für R sind C₃-C₅-Alkyl, C₂-C₅-Alkenyl, C₂-C₄-Alkinyl, Benzyl, Phenethyl, Phenyl, durch C₁-C₃-Alkyl, C₁-C₃-Alkoxy, C₁-C₃-Alkylmercapto, Allyl, Allyloxy, C₁-C₃-Alkylamino, Di-C₁-C₃-alkylamino, Amino, Hydroxy, Azido, Trifluormethyl, Cyano oder Halogen mono- oder disubstituiertes Phenyl bzw. durch Methyl oder Halogen trisubstituiertes Phenyl, Naphthyl, Anthracenyl, Indenyl, Acenaphthylenyl, Phenanthrenyl, Adamantyl, Cyclohexyl, Cyclohexenyl, Furyl, Thienyl, Pyridyl, Pyrimidinyl, Thiazolyl, Indolyl, Chinolinyl, Benzimidazolyl, Methylendioxyphenyl, Carbazolyl und Phenothiazinyl und durch Methyl oder Halogen mono- oder disubstituierte Derivate der vorgenannten carbocyclischen oder heterocyclischen Ringe.

Für R¹ ist besonders bevorzugt Wasserstoff, Methyl, Ethyl, Isopropyl, Allyl, Methoxy, Ethoxy, Methylmercapto, Ethylmercapto, Methylamino, Methoxycarbonyl, Ethoxycarbonyl, Amino, Azido, Cyano, Hydroxy und Halogen, wobei Chlor und Brom für Halogen bevorzugt in Frage kommen.

Für R², R³, R⁴ und R⁵ sind Wasserstoff, Methyl, Ethyl, Isopropyl und Cyano besonders bevorzugt oder wenn R² und R⁴ eine zusätzliche Bindung bedeuten. Insbesondere kommen solche Verbindungen der Formel I in Frage, bei denen R² und R⁴ gleichzeitig Wasserstoff bedeuten.

Insbesondere bevorzugt für A sind aromatische Ringe, wie z.B. der Phenyl-, Pyrrol-, Pyrrol-, Oxazol-, Thiophen-, Furan-, Isoxazol-, Thiazol-, Imidazol-, Pyridin-, Pyridazin-, Pyrimidin- und Pyrazinring. Für den Fall, daß A einen carbocyclischen oder heterocylischen Sechsring darstellt, sind insbesondere solche Verbindungen bevorzugt, die in bezug auf das direkt an den Sechsring A gebundene Stickstoffatom des ankondensierten Pyrimidinringes in para-Stellung des Sechsringes A (9-Position des tricyclischen Ringsystems) substituiert sind.

Insbesondere bevorzugt sind Verbindungen der allgemeinen Formel I, in denen R, R¹, X, n und m die oben angegebene Bedeutung haben, R², R³, R⁴ und R⁵ gleich Wasserstoff, Methyl oder Ethyl, sind, wobei drei oder vier der Reste R² bis R⁵ bevorzugt Wasserstoff bzw. R² und R⁴ gemeinsam eine Bindung darstellen.

Für R⁶ ist insbesondere Wasserstoff und C₁-C₃-Alkyl bevorzugt. Für n und m ist insbesondere O bevorzugt.

Verbindungen der Formel I, in denen R¹ ein Wasserstoff- oder Halogenatom, insbesondere ein Chloratom, A einen Phenyl- oder Pyridinring, der vorzugsweise in der 9-Position des tricyclischen Ringsystems durch R¹ substituiert ist, besitzen eine besonders gut ausgeprägte pharmakologische Wirkung. Besonders bevorzugt kommen dabei solche Verbindungen der Formel I in Frage, bei denen R²-R⁵ ein Wasserstoffatom, m und n die Zahl 0 und X ein Sauerstoff- oder Schwefelatom bedeuten.

Die Arzneimittel enthalten mindestens eine Verbindung der Formel I zur Behandlung von viralen Infektionen und können in flüssiger oder fester Form enteral oder parenteral appliziert werden. Als pharmazeutische Darreichungsformen kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die Verbindungen der vorliegenden Erfindung und ihre pharmazeutischen Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind, wie 3'-Azido-3'desoxythymidin, 2',3'-Didesoxynukleoside wie z. B. 2',3'-Didesoxycytidin, 2',3'-Didesoxyadenosin und 2',3'-Didesoxyinosin oder acyclische Nukleoside (z. B. Acyclovir).

Die Verbindungen der vorliegenden Erfindung und das andere Arzneimittel können jeweils einzeln, gleichzeitig, gegebenenfalls in einer einzigen oder zwei getrennten Formulierungen oder zu unterschiedlichen Zeiten verabreicht werden, so daß ein synergistischer Effekt erreicht wird.

Die erfindungsgemäßen Verbindungen der allgemeinen Formel I werden nach literaturbekannten Verfahren (Chem. Pharm. Bull. 29, 2135, 1981 oder Heterocycles 29, 1317, 1989) hergestellt, indem man Verbindungen der allgemeinen Formel II 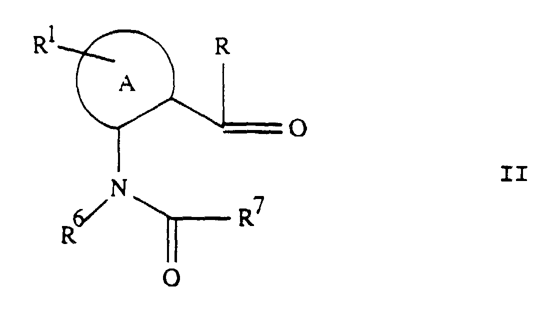 in der A, R, R¹ und R⁶ die oben angegebene Bedeutung haben und R⁷ eine leicht abspaltbare Gruppe, wie CCl₃, CF₃, OR⁸ oder NR⁸R⁹, wobei R⁸ und R⁹ gleich oder verschieden sein können und beispielsweise C₁-C₆-Alkyl oder substituiertes oder unsubstituiertes Phenyl, Phenylalkyl, Hetaryl oder Hetarylalkyl bedeuten können, mit substituiertem oder unsubstituiertem Cysteamin bzw. 3-Mercapto-1-propanamin der allgemeinen Formel III 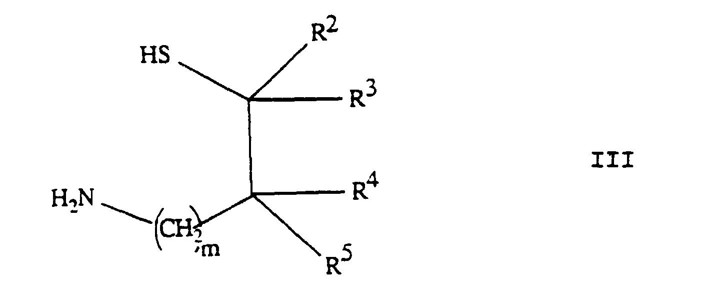 in der R², R³, R⁴, R⁵ und m die angegebene Bedeutung haben, in einem geeigneten inerten Lösungsmittel bei Raumtemperatur bis Rückflußtemperatur evtl. in Gegenwart katalytischer Mengen Säure, z.B. p-Toluolsulfonsäure, oder Base z.B. Kaliumhydroxyd umsetzt und gegebenenfalls anschließend Verbindungen der Formel I in andere Verbindungen der Formel I nachträglich umwandelt und anschließend chromatographisch bzw. durch Umkristallisation reinigt. Racemate können durch Chromatographie an geeigneten optisch aktiven Phasen, z.B. Cellulosetriacetat, in die Antipoden getrennt werden.

Die nachträglichen Umwandlungen von Verbindungen der Formel I in andere Verbindungen der Formel I betreffen z.B. die Herstellung von tricyclischen Thiazolo[3,2-c]pyrimidin- und Thiazino[3,2-c]pyrimidin-Derivaten mit X=S. Verbindungen mit X=S werden hergestellt durch Umsetzung von Verbindungen der Formel I, in der X ein Sauerstoffatom bedeutet, mit schwefelgruppenübertragenden Verbindungen, wie z.B. Lawesson's Reagenz.

Verbindungen der allgemeinen Formel I mit X = NH werden hergestellt, durch Umsetzung von Verbindungen der Formel I, in der X ein Sauerstoffatom bedeutet, indem man mit einem Chlorierungsreagenz, wie z.B. POCl₃, zunächst das Chlorimin herstellt und dieses mit Ammoniak behandelt.

Verbindungen der allgemeinen Formel I, in der R² und R⁴ eine zusätzliche Bindung bedeuten, werden hergestellt indem man in einer Pummerer-Reaktion Verbindungen der allgemeinen Formel I mit n = 1 mit einem Anhydrid unter evt. Erwärmung in einem inerten Lösungsmittel umsetzt, oder Verbindungen der allgemeinen Formel I, in der R², R³, R⁴ oder R⁵ einen mit einer Carbonsäure, wie z. B. Essigsäure, oder Sulfonsäure, wie z. B. Toluolsulfonsäure, veresterten Hydroxymethylrest bedeutet, in einem inerten Lösungsmittel in Gegenwart einer Base, wie z. B. Imidazol oder Natriumhydroxid, auf 50-200°C evt. unter Druck erwärmt.

Die als Ausgangsmaterial verwendeten Verbindungen der allgemeinen Formel II werden nach literaturbekannten Verfahren aus Aminobenzophenon-Derivaten durch Acylierung gewonnen. Die substituierten oder unsubstituierten 2-Aminobenzophenon-Derivate lassen sich vorteilhaft nach den von David A. Walsh beschriebenen Verfahren (Synthesis, 677, 1980) herstellen.

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die als racemischen Gemische oder in optisch aktiver Form bzw. als reine R- und S-Enantiomere vorliegen können:
1. 10b-Phenyl-2,3,6,10b-tetrahydro-5H-thiazolo[3,2-c]quinazolin-5-on
2. 10b-Phenyl-2,3,6,10b-tetrahydro-5H-thiazolo[3,2-c]quinazolin-5-thion
3. 10b-Phenyl-2,3,6,10b-tetrahydro-5H-thiazolo[3,2-c]quinazolin-5-imin
4. 6-Methyl-10b-phenyl-2,3,6,10b-tetrahydro-5H-thiazolo[3,2-c]quinazolin-5-on
5. 9-Chlor-10b-phenyl-2,3,6,10b-tetrahydro-5H-thiazolo[3,2-c]quinazolin-5-on
6. 9-Methyl-10b-phenyl-2,3,6,10b-tetrahydro-5H-thiazolo[3,2-c]quinazolin-5-on
7. 9-Methoxy-10b-phenyl-2,3,6,10b-tetrahydro-5H-thiazolo[3,2-c]quinazolin-5-on
8. 10-Chlor-10b-phenyl-2,3,6,10b-tetrahydro-5H-thiazolo[3,2-c]quinazolin-5-on
9. 10-Nitro-10b-phenyl-2,3,6,10b-tetrahydro-5H-thiazolo[3,2-c]quinazolin-5-on
10. 7-Methyl-10b-phenyl-2,3,6,10b-tetrahydro-5H-thiazolo[3,2-c]quinazolin-5-on
11. 8-Methyl-10b-phenyl-2,3,6,10b-tetrahydro-5H-thiazolo[3,2-c]quinazolin-5-on
12. 10b-(3-Methylphenyl)-2,3,6,10b-tetrahydro-5H-thiazolo[3,2-c]quinazolin-5-on
13. 10b-(3-Chlorphenyl)-2,3,6,10b-tetrahydro-5H-thiazolo[3,2-c]quinazolin-5-on
14. 10b-(4-Methoxyphenyl)-2,3,6,10b-tetrahydro-5H-thiazolo[3,2-c]quinazolin-5-on
15. 10b-(2,3-Dimethylphenyl)-2,3,6,10b-tetrahydro-5H-thiazolo[3,2-c]quinazolin-5-on
16. 10b-(3,5-Dimethylphenyl)-2,3,6,10b-tetrahydro-5H-thiazolo[3,2-c]quinazolin-5-on
17. 10b-(1-Naphthyl)-2,3,6,10b-tetrahydro-5H-thiazolo[3,2-c]quinazolin-5-on
18. 10b-(4-Indanyl)-2,3,6,10b-tetrahydro-5H-thiazolo[3,2-c]quinazolin-5-on
19. 10b-(2-Amino-5-methylphenyl)-2,3,6,10b-tetrahydro-5H-thiazolo[3,2-c]quinazolin-5-on
20. 10b-(6-Methyl-2-pyridyl)-2,3,6,10b-tetrahydro-5H-thiazolo[3,2-c]quinazolin-5-on
21. 10b-(4-Methyl-2-pyridyl)-2,3,6,10b-tetrahydro-5H-thiazolo[3,2-c]quinazolin-5-on
22. 10b-Thienyl-2,3,6,10b-tetrahydro-5H-thiazolo[3,2-c]quinazolin-5-on
23. 10b-Furanyl-2,3,6,10b-tetrahydro-5H-thiazolo[3,2-c]quinazolin-5-on
24. 11b-Phenyl-3,4,7,11b-tetrahydro-2H,6H-[1,3]thiazino[3,2-c]quinazolin-6-on
25. 10b-Phenyl-2,3,6,10b-tetrahydro-5H-thiazolo[3,2-c]pyrido[2,3-e]pyrimidin-5-on
26. 10b-(3-Methylphenyl)-2,3,6,10b-tetrahydro-5H-thiazolo[3,2-c]pyrido[2,3-e]pyrimidin-5-on
27. 10b-(3-Chlorphenyl)-2,3,6,10b-tetrahydro-5H-thiazolo[3,2-c]pyrido[2,3-e]pyrimidin-5-on
28. 10b-(6-Methyl-2-pyridyl)-2,3,6,10b-tetrahydro-5H-thiazolo[3,2-c]pyrido[2,3-e]pyrimidin-5-on
29. 10b-(4-Methyl-2-pyridyl)-2,3,6,10b-tetrahydro-5H-thiazolo[3,2-c]pyrido[2,3-e]pyrimidin-5-on
30. 3-Ethoxycarbonyl-10b-phenyl-2,3,6,10b-tetrahydro-5H-thiazolo[3,2-c]pyrido[2,3-e]pyrimidin-5-on
31. 3-Hydroxymethyl-10b-phenyl-2,3,6,10b-tetrahydro-5H-thiazolo[3,2-c]pyrido[2,3-e]pyrimidin-5-on
32. 10b-Phenyl-2,3,6,10b-tetrahydro-5H-thiazolo[3,2-c]-pyrido[3,4-e]pyrimidin-5-on
33. 10b-(3-Ethylphenyl)-2,3,6,10b-tetrahydro-5H-thiazolo[3,2-c]pyrido[3,4-e]pyrimidin-5-on
34. 10b-(3-Nitrophenyl)-2,3,6,10b-tetrahydro-5H-thiazolo[3,2-c]pyrido[3,4-e]pyrimidin-5-on
35. 10b-(6-Methyl-2-pyridyl)-2,3,6,10b-tetrahydro-5H-thiazolo[3,2-c]pyrido[3,4-e]pyrimidin-5-on
36. 10b-Methyl-2,3,6,10b-tetrahydro-5H-thiazolo[3,2-c]-pyrido[3,4-e]pyrimidin-5-on
37. 9b-Phenyl-2,3,6,9b-tetrahydro-5H-thiazolo[3,2-c]-thieno[2,3-e]pyrimidin-5-on
38. 9b-(3-Methylphenyl)-2,3,6,9b-tetrahydro-5H-thiazolo[3,2-c]thieno[2,3-e]pyrimidin-5-on
39. 9b-(3,5-Dichlorphenyl)-2,3,6,9b-tetrahydro-5H-thiazolo[3,2-c]thieno[2,3-e]pyrimidin-5-on
40. 9b-Phenyl-2,3,6,9b-tetrahydro-5H-thiazolo[3,2-c]furano[2,3-e]pyrimidin-5-on
41. 9b-(4-Methyl-2-pyridyl)-2,3,6,9b-tetrahydro-5H-thiazolo[3,2-c]furano[2,3-e]pyrimidin-5-on
42. 10b-Phenyl-2,3,6,10b-tetrahydro-5H-thiazolo[3,2-c]quinazolin-5-on-1-oxid
43. 10b-Phenyl-2,3,6,10b-tetrahydro-5H-thiazolo[3,2-c]quinazolin-5-on-1,1-dioxid
44. 10b-Phenyl-6,10-dihydro-5H-thiazolo[3,2-c]quinazolin-5-on
45. 10b-(3-Methylphenyl)-6,10-dihydro-5H-thiazolo[3,2-c]quinazolin-5-on
46. 3-Methyl-10b-phenyl-6,10-dihydro-5H-thiazolo[3,2-c]quinazolin-5-on
47. 10b-(3-Methylphenyl)-6,10-dihydro-5H-thiazolo[3,2-c]pyrido[2,3-e]pyrimidin-5-on
48. 3-Methyl-10b-(3-methylphenyl)-6,10-dihydro-5H-thiazolo[3,2-c]pyrido[2,3-e]pyrimidin-5-on

### Beispiel 1

### 10b-Phenyl-2,3,6,10-tetrahydro-5H-thiazolo[3,2-c]quinazolin-5-on

a) Zu 10 g (50.7 mmol) 2-Aminobenzophenon in 125 ml Toluol und 4.9 ml Pyridin tropft man 5.8 ml (60.8 mmol) Chlorameisensäureethylester in 25 ml Toluol bei 10°C unter Rühren. Nach einer Stunde wird der Ansatz 3 x mit Wasser geschüttelt, die organische Phase abgetrennt, getrocknet und eingedampft. Das erhaltene Öl wird mit Isohexan angerieben und die Kristalle abgesaugt. Man erhält 12.3 g 2-Ethoxycarbonylaminobenzophenon vom Smp. 73-75°C.
b) 5 g (18.6 mmol) 2-Ethoxycarbonylaminobenzophenon, 2.9 g (37.1 mmol) Cysteamin und 0.5 g p-Toluolsulfonsäure werden in 100 ml Dimethylformamid (DMF) 52 Stunden unter Stickstoff auf 140°C erhitzt. Anschließend wird das DMF abdestilliert, der Rückstand in Wasser gelöst und 2 x mit Dichlormethan ausgeschüttelt. Die organische Phase wurde getrocknet, eingedampft und der Rückstand über Kieselgel (Laufmittel: Isohexan:Essigester, 7:3) chromatographiert. Eindampfen der gewünschten Fraktionen und Umkristallisation des Rückstandes aus Ethanol liefert 3.5 g der Titelverbindung vom Smp. 212-215°C.

### Beispiel 2

### 11b-Phenyl-3,4,7,11b-tetrahydro-2H,6H-[1,3]thiazino[3,2-c]quinazolin-6-on

5 g (18.6 mmol) 2-Ethoxycarbonylaminobenzophenon (aus Bsp. 1a), 3.4 g (37.1 mmol) 3-Mercapto-1-propanamin und 0.5 g p-Toluolsulfonsäure werden in 100 ml Dimethylformamid (DMF) 52 Stunden unter Stickstoff auf 140° C erhitzt. AnschlieBend wird das DMF abdestilliert, der Rückstand in Wasser gelöst und 2 x mit Dichlormethan ausgeschüttelt. Die organische Phase wurde getrocknet, eingedampft und der Rückstand über Kieselgel (Laufmittel : Isohexan : Essigester, 7:3) chromatographiert. Eindampfen der gewünschten Fraktionen und Umkristallisation des Rückstandes aus Ethanol liefert 3.2 g der Titelverbindung vom Smp. 240-243° C.

### Beispiel 3

### 3-Hydroxymethyl-10b-phenyl-2,3,6,10-tetrahydro-5H-thiozolo [3,2-c]quinazolin-5-on

a) Analog Beispiel 1 a setzt man 10 g (50.7 mmol) 2-Aminobenzophenon mit 8.6 (55 mmol) Chlorameisensäurephenylester um. Nach Aufarbeitung und Anreiben mit Isohexan erhält man 15.6 g 2-Phenoxycarbonylaminobenzphenon mit Smp. 115-117°C.
b) 22.19 g (70 mmol) 2-Phenoxycarbonylaminobenzophenon, 22 g (210 mmol) Cysteinol und 2.2 g p-Toluolsulfonsäure wurden in 500 ml Toluol 24 Stunden unter Stickstoff am Rückfluß erhitzt. Anschließend wurde das Toluol abdestilliert und das Öl säulenchromatgraphisch (Laufmittel: Essigester/ Toluol = 80:20) gereinigt. Die vereinigten Fraktionen der gewünschten Substanz wurden eingeengt und der Rückstand aus Toluol kristallisiert. Man erhält 3.5 g der Titelverbindung mit Spm. 188-190°C.

### Beispiel 4

### 9-Chlor-10b-phenyl-2,3,6,10b-tetrahydro-5H-thiazolo[3,2-c]quinazolin-5-on

Analog Beispiel 3 b wurden 3.0 g (8.5 mmol) 2-Phenoxycarbonylamino-5-chlorbenzophenon, 1.3 g (17 mmol) Cysteamin und 0.3 g p-Toluolsulfonsäure in 150 ml Toluol 4 Stunden am Wasserabscheider gekocht. Die Toluolphase wurde mit 2 N Salzsäure und Natriumkarbonatlösung geschüttelt, eingeengt und der Rückstand aus Ethanol kristallisiert. Man erhält 2.1 g der Titelverbindung mit Smp. 187-194°C.

### Beispiel 5

### Analog Beispiel 4 erhält man die folgenden Verbindungen:

5 a) 10-Chlor-10b-phenyl-2,3,6,10b-tetrahydro-5H-thiazolo[3,2-c]quinazolin-5-on mit Smp. 264-265°C aus Ethanol
5 b) 10b-(3-Methylphenyl)-2,3,6,10b-tetrahydro-5H-thiazolo[3,2-c]quinazolin-5-on mit Smp. 236-240°C aus Ethanol.
5 c) 10b-(3-Chlorphenyl)-2,3,6,10b-tetrahydro-5H-thiazolo[3,2-c]quinazolin-5-on mit Smp. 226-227°C aus Ethanol.
5 d) 10b-(3,5-Dimethylphenyl)-2,3,6,10b-tetrahydro-5H-thiazolo[3,2-c]quinazolin-5-on mit Smp. 243-256°C aus Ethanol.
5 e) 10b-(3,5-Dichlorphenyl)-2,3,6,10b-tetrahydro-5H-thiazolo[3,2-c]quinazolin-5-on mit Smp. 260-261°C aus Ethanol.
5 f) 10b-(6-Methyl-2-pyridyl)-2,3,6,10b-tetrahydro-5H-thiazolo[3,2-c]quinazolin-5-on mit Smp. 260-261°C aus Ethanol.

### Beispiel 6

### 6-Methyl-10b-phenyl-2,3,6,10b-tetrahydro-5H-thiazolo [3,2-c]quinazolin-5-on

0.5 g (1.8 mmol) der in Beispiel 1 b erhaltenen Verbindung wurden in 10 ml DMF gelöst, mit 65 mg (2.7 mmol) NaH und 0.14 ml (2.2 mmol) Methyljodid versetzt. Nach 2 Stunden bei 25°C wurde das DMF abdestilliert, der Rückstand in Dichlormethan aufgenommen, mit Wasser gewaschen und die organische Phase getrocknet und eingeengt. Nach Kirstallisation aus Ethanol erhält man 0.4 g der Titelverbindung mit Smp. 172-174°C.

### Beispiel 7

### 10b-Phenyl-2,3,6,10b-tetrahydro-5H-thiazolo[3,2-c]quinazolin-5-thion

3.0 g (11 mmol) der in Beispiel 1 b erhaltenen Verbindung und 8.8 g (22 mmol) Lawesson-Reagenz wurden in 150 ml Toluol 16 Stunden zum Rückfluß erhitzt. Anschließend wurde mit Wasser versetzt, vom Ungelösten abgetrennt und die organische Phase getrocknet und eingeengt. Der Rückstand wurde über Kieselgel (Laufmittel: Essigester/Isohexan, 40:60) gereinigt. Nach Kristallisation aus Ethanol erhält man 1.5 g der Titelverbindung mit Smp. 208-215°C.

### Beispiel 8

### 10b-Phenyl-2,3,6,10b-tetrahydro-5H-thiazolo[312-c]quinazolin-5-imin

a) 0.25 g (0.84 mmol) der in Bsp. 7 erhaltenen Verbindung wurde in 5 ml DMF mit 31 mg NaH und 0.06 ml Methyliodid versetzt. Nach 1 Stunde wurde das DMF abdestilliert, mit Methylenchlorid und Wasser versetzt, angesäuert auf pH 5 und die organische Phase abgetrennt. Nach Trocknen und Eindampfen erhält man 250 mg 10b-phenyl-5-methylmercapto-2,3-dihydrothiazolo[3,2-c]quinazolin als Öl, R_{f}=0.9 (Toluol/Dioxan/Wasser).
b) 250 mg der vorangegangenen Verbindung wurden mit 15 ml Ethanol und 10 ml flüssigem Ammoniak im Autoklaven 9 Stunden bei 80°C geschüttelt. Nach dem Eindampfen wurde der Rückstand über Kieselgel (Laufmittel: 80 % Essigester - 20 % Isohexan) chromatographiert. Nach Eindampfen der gewünschten Fraktionen und Kristallisation aus Essigester erhält man 75 mg der Titelverbindung von Smp. 200-204°C.

### Beispiel 9

### Hemmung der Reversen Transkriptase (RT)

Das Screeningtestsystem beinhaltet die gereinigte RT aus HIV-1, die durch gentechnologische Methoden in E. coli exprimiert wurde, sowie die Komponenten des Initiationskomplexes, wie die in-vitro-Transkripte des HIV-LTR's mit der benachbarten Primer Binding Site als Template und einem zur Primer Binding Site komplementären 18mer Oligonukleotid als Primer. Gemessen wurde der [³H]-Thymidin-5'-triphosphat-Einbau durch Auszählen im β-Counter.

**Ergebnisse:**

| **Beispiel** | **Hemmung der HIV-RT IC**_{**50**}**[µM]** |
|---|---|
| 1 b | 0.47 |
| 2 | 0.51 |
| 7 | 0.03 |
| 4 | 0.03 |
| 6 | 6.20 |
| 5 b | 0.03 |
| 5 f | 0.20 |

## Patentansprüche

1. Tricyclische Thiazolo[3,2-c]pyrimidin- und Thiazino[3,2-c]pyrimidin-Derivate der Formel I 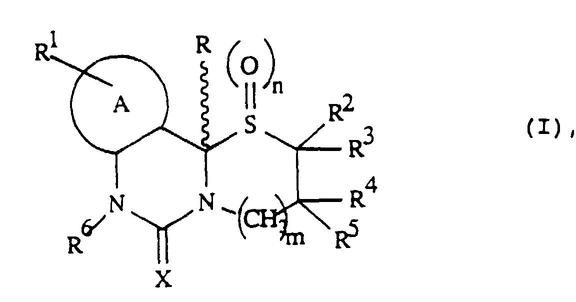 in der
A einen carbocyclischen Ring mit 5 oder 6 Kohlenstoffatomen oder einen heterocyclischen Ring mit max. 4 Heteroatomen darstellt, wobei die Heteroatome gleich oder verschieden sein können und Sauerstoff, Stickstoff oder Schwefel bedeuten, und die Heterocyclen gegebenenfalls an einem oder mehreren Stickstoffatomen ein Sauerstoffatom tragen können, und A gegebenenfalls substituiert ist durch einen oder mehrere Reste R¹, die gleich oder verschieden sein können,
X ein Sauerstoff- oder Schwefelatom oder die Gruppe =NH, =N-C₁-C₆-Alkyl oder =S(O)₂ sein kann,
R einen geradkettigen oder verzweigten, gesättigten oder ungesättigten aliphatischen Rest mit 1-9 C-Atomen, der durch Phenyl substituiert sein kann, oder
einen Phenylring bedeutet,
oder einen mono-, bi- oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können, wobei die Heteroatome Stickstoff, Schwefel oder Sauerstoff sind, und die vorgenannten Phenylringe, die mono-, bi- oder tricyclischen carbocyclischen Ringe oder das heterocyclische mono-, bi- oder tricyclische Ringsystem gegebenenfalls ein- oder mehrfach substituiert ist durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylamino-carbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl,
R¹ ein Wasserstoffatom, einen geradkettigen oder verzweigten, gesättigten oder ungesättigten aliphatischen Rest mit 1-6 C-Atomen oder C₁-C₆-Alkoxy, C₁-C₆-Alkylmercapto, C₁-C₆-Alkylsulfinyl, C₁-C₆-Alkylsulfonyl, Amino, C₁-C₆-Alkylamino, Di-C₁-C₆-Alkylamino, Sulfonamido, C₁-C₆-Alkoxycarbonyl, Carboxy, Halogen, Hydroxy, Nitro, Cyano, Azido, Phenyl oder Benzyloxy bedeutet,
R², R³, R⁴ und R⁵ unabhängig voneinander gleich oder verschieden sein können und jeweils Wasserstoff, C₁-C₆-Alkyl, C₁-C₆-Hydroxyalkyl, Cyano, Carboxy oder C₁-C₆-Alkoxycarbonyl oder R² und R⁴ eine weitere Bindung zwischen den C-Atomen, an die sie gebunden sind, bedeuten können,
R⁶ Wasserstoff oder C₁-C₆-Alkyl bedeutet,
n 0, 1 oder 2 bedeutet und
m 0 oder 1 bedeutet,
sowie deren Tautomere, Enantiomere, Diastereomere und physiologisch verträgliche Salze.

2. Tricyclische Thiazolo[3,2-c]pyrimidin- und Thiazino[3,2-c]pyrimidin-Derivate gemäß Anspruch 1, dadurch gekennzeichnet, daß A einen einfach durch R¹ substituierten Phenylring oder einen heterocyclischen Ring ausgewählt aus der Gruppe Furanyl-, Thiophenyl-, Pyrrolyl-, Oxazolyl-, Isoxazolyl-, Thia-zolyl-, Pyrazolyl-, Imidazolyl-, Oxadiazolyl-, Triazolyl-, Pyridinyl-, Pyridazinyl-, Pyrimidinyl- oder Pyrazinylring bedeutet.

3. Tricyclische Thiazolo[3,2-c]pyrimidin- und Thiazino[3,2-c]pyrimidin-Derivate gemäß Anspruch 1 oder 2, dadurch gekennzeichnet, daß R eine Phenylgruppe oder einen carbocyclischen Ring aus der Gruppe Naphthyl, Anthracenyl, Phenanthrenyl, Flourenyl, Indenyl, Indanyl, Acenaphthylenyl, Norbornyl, Adamantyl, C₃-C₇-Cycloalkyl oder C₅-C₈-Cycloalkenyl bedeutet, wobei diese Gruppen unsubstituiert oder substituiert sein können durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₃-C₆-Alkenyloxy, C₁-C₆-Alkylamino, C₁-C₆-Dialkylamino-, C₁-C₆-Alkylcarbonylamino, C₁-C₆-Alkylaminocarbonyl, C₁-C₆-Alkoxycarbonyl-, Amino, Hydroxy, Nitro, Azido, Trifluormethyl, Cyano oder Halogen.

4. Tricyclische Thiazolo[3,2-c]pyrimidin- und Thiazino[3,2-c]pyrimidin-Derivate gemäß Anspruch 1 oder 2, dadurch gekennzeichnet, daß R einen heterocyclischen Rest bedeutet, ausgewählt aus der Gruppe Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei diese Heterocyclen partiell oder vollständig hydriert und unsubstituiert oder substituiert sein können durch C₁-C₃-Alkyl, C₁-C₃-Alkoxy, C₁-C₃-Alkylmercapto, C₁-C₃-Alkylsulfinyl, C₁-C₃-Alkylsulfonyl, C₂-C₄-Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

5. Tricyclische Thiazolo[3,2-c]pyrimidin- und Thiazino[3,2-c]pyrimidin-Derivate gemäß den Ansprüchen 1-4, dadurch gekennzeichnet, daß R¹ Wasserstoff, C₁-C₃-Alkyl, C₂-C₄-Alkenyl, C₂-C₄-Alkinyl, C₁-C₃-Alkoxy, C₁-C₃-Alkylmercapto, C₁-C₃-Alkylamino, C₁-C₃-Alkoxycarbonyl, Amino, Halogen, Hydroxy, Nitro, Cyano und Azido bedeutet.

6. Tricyclische Thiazolo[3,2-c]pyrimidin- und Thiazino[3,2-c]pyrimidin-Derivate gemäß den Ansprüchen 1-5, dadurch gekennzeichnet, daß A einen Phenyl- oder Pyridinylring bedeutet, der durch ein Halogenatom substituiert sein kann, R²-R⁶ jeweils ein Wasserstoffatom, m und n die Zahl 0 und X ein Sauerstoff- oder Schwefelatom darstellen.

7. Verfahren zur Herstellung von tricyclischen Thiazolo[3,2-c]pyrimidin- und Thiazino-[3,2-c]pyrimidin-Derivaten gemäß den Ansprüchen 1-6, dadurch gekennzeichnet, daß man Verbindungen der allgemeinen Formel II 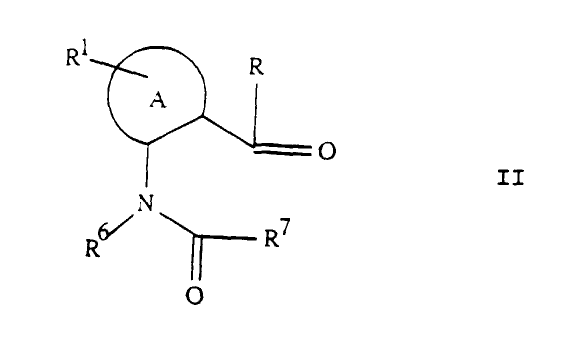 in der A, R, R¹ und R⁶ die oben angegebene Bedeutung haben und R⁷ eine leicht abspaltbare Gruppe, wie CCl₃, CF₃, OR⁸ oder NR⁸R⁹, wobei R⁸ und R⁹ gleich oder verschieden sein können und beispielsweise C₁-C₆-Alkyl oder substituiertes oder unsubstituiertes Phenyl, Phenylalkyl, Hetaryl oder Hetarylalkyl bedeuten können, mit substituiertem oder unsubstituiertem Cysteamin bzw. 3-Mercapto-1-propanamin der allgemeinen Formel III 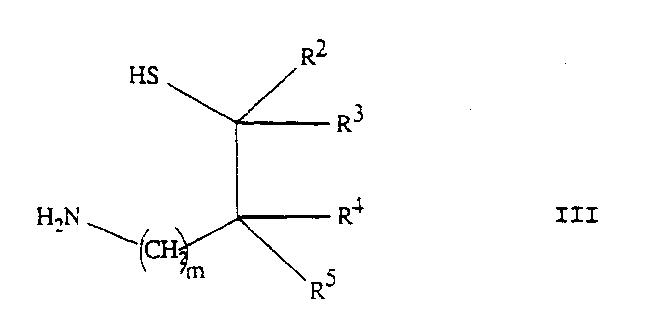 in der R², R³, R⁴, R⁵ und m die angegebene Bedeutung haben, in einem geeigneten inerten Lösungsmittel bei Raumtemperatur bis Rückflußtemperatur evtl. in Gegenwart katalytischer Mengen Säure oder Base umsetzt und gegebenenfalls anschließend Verbindungen der Formel I mit X=0 in Verbindungen der Formel I mit X=S oder X=NH nachträglich umwandelt.

8. Arzneimittel enthaltend mindestens ein tricyclisches Thiazolo[3,2-c]pyrimidin- oder Thiazino-[3,2-c]pyrimidin-Derivat der Formel I gemäß den Ansprüchen 1-6, sowie pharmakologisch verträgliche Hilfs- oder Trägerstoffe.

9. Verwendung von tricyclischen Thiazolo[3,2-c]pyrimidin- oder Thiazino-[3,2-c]pyrimidin-Derivaten gemäß den Ansprüchen 1-6 zur Herstellung von Arzneimitteln mit antiviraler Wirkung.

10. Verfahren zur Herstellung von Arzneimitteln gemäß Anspruch 7, dadurch gekennzeichnet, daß man mindestens eine Verbindung der Formel I zusammen mit pharmakologisch verträglichen Hilfsstoffen mischt und zu pharmazeutischen Darreichungsformen verarbeitet.

## Claims

1. Tricyclic thiazolo[3,2-c] pyrimidine and thiazino[3,2-c]pyrimidine derivatives of formula I 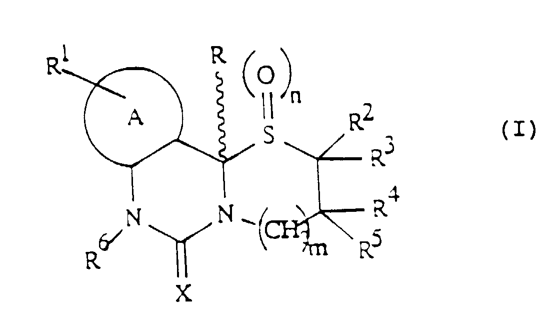 in which
A represents a carbocyclic ring with 5 or 6 carbon atoms or a heterocyclic ring with a maximum of 4 heteroatoms in which the heteroatoms can be the same or different and denote oxygen, nitrogen or sulphur and, if desired, the heterocycles can carry an oxygen atom on one or several nitrogen atoms, and A is substituted, if desired, by one or several residues R¹ which can be the same or different,
x can be an oxygen or sulphur atom or the group =NH, =N-C₁-C₆-alkyl or =S(O)₂,
R can be a straight-chained or branched, saturated or unsaturated aliphatic residue with 1-9 C atoms which can be substituted by phenyl or
it denotes a phenyl ring,
or R denotes a mono-, bi- or tricyclic carbocyclic ring with 7-15 C atoms or a heterocyclic mono-, bi- or tricyclic ring system with 5 or 6 ring atoms in each case and can contain 1-4 or 1-5 heteroatoms per ring system in which the heteroatoms are nitrogen, sulphur or oxygen and the aforementioned phenyl rings, the mono-, bi- or tricyclic carbocyclic rings or the heterocyclic mono-, bi- or tricyclic ring system is substituted, if desired, once or several times by C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₂-C₆ alkenyloxy, C₂-C₆ alkenylmercapto, C₂-C₆ alkinyloxy, C₂-C₆ alkinylmercapto, amino, C₁-C₆ alkylamino, di-C₁-C₆-alkylamino, C₁-C₆ alkylcarbonylamino, C₁-C₆ alkylaminocarbonyl, C₁-C₆ alkoxycarbonyl, hydroxy, benzyloxy, phenylmercapto, phenyloxy, nitro, cyano, halogen, trifluoromethyl, azido, formylamino, carboxy or phenyl,
R¹ denotes a hydrogen atom, a straight-chained or branched, saturated or unsaturated aliphatic residue with 1-6 C atoms or C₁-C₆ alkoxy, C₁-C₆ alkylmercapto, C₁-C₆ alkylsulfinyl, C₁-C₆ alkylsulfonyl, amino, C₁-C₆ alkylamino, di-C₁-C₆₋ alkylamino, sulfonamido, C₁-C₆ alkoxycarbonyl, carboxy, halogen, hydroxy, nitro, cyano, azido, phenyl or benzyloxy,
R², R³, R⁴ and R⁵ can, independently of each other, be the same or different and each denote hydrogen, C₁-C₆ alkyl, C₁-C₆ hydroxyalkyl, cyano, carboxy or C₁-C₆ alkoxycarbonyl or R² and R⁴ can denote a further bond between the C atoms to which they are bound,
R⁶ denotes hydrogen or C₁-C₆ alkyl,
n denotes 0, 1 or 2 and
m denotes 0 or 1
as well as tautomers, enantiomers, diastereomers and physiologically tolerated salts thereof.

2. Tricyclic thiazolo[3,2-c]pyrimidine and thiazino[3,2-c]pyrimidine derivatives as claimed in claim 1, wherein A denotes a single phenyl ring substituted by R¹ or a heterocyclic ring selected from the group furanyl, thiophenyl, pyrrolyl, oxazolyl, isoxazolyl, thiazolyl, pyrazolyl, imidazolyl, oxadiazolyl, triazolyl, pyridinyl, pyridazinyl, pyrimidinyl or pyrazinyl ring.

3. Tricyclic thiazolo[3,2-c]pyrimidine and thiazino[3,2-c]pyrimidine derivatives as claimed in claim 1 or 2, wherein R denotes a phenyl group or a carbocyclic ring from the group naphthyl, anthracenyl, phenanthrenyl, flourenyl, indenyl, indanyl, acenaphthylenyl, norbornyl, adamantyl, C₃-C₇ cycloalkyl or C₅-C₈ cycloalkenyl and these groups can be unsubstituted or substituted by C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₃-C₆ alkenyloxy, C₁-C₆ alkylamino, C₁-C₆ dialkylamino, C₁-C₆ alkyl-carbonylamino, C₁-C₆ alkylaminocarbonyl, C₁-C₆ alkoxycarbonyl, amino, hydroxy, nitro, azido, trifluoromethyl, cyano or halogen.

4. Tricyclic thiazolo[3,2-c]pyrimidine and thiazino[3,2-c]pyrimidine derivatives as claimed in claim 1 or 2, wherein R denotes a heterocyclic residue selected from the group pyridine, pyrimidine, pyridazine, pyrazine, triazine, pyrrole, pyrazole, imidazole, triazole, thiazole, oxazole, isoxazole, oxadiazole, furazan, furan, thiophene, indole, quinoline, isoquinoline, coumarone, thionaphthene, benzoxazole, benzthiazole, indazole, benzimidazole, benztriazole, chromene, phthalazine, quinazoline, quinoxaline, methylenedioxybenzene, carbazole, acridine, phenoxazine, phenothiazine, phenazine or purine system in which these heterocycles can be partially or completely hydrogenated and unsubstituted or substituted by C₁-C₃ alkyl, C₁-C₃ alkoxy, C₁-C₃ alkylmercapto, C₁-C₃ alkylsulfinyl, C₁-C₃ alkylsulfonyl, C₂-C₄ alkenyl, C₂-C₃ alkinyl, C₃-C₄ alkenyloxy, C₁-C₃ alkylamino, C₁-C₃ dialkylamino, C₁-C₃ alkylcarbonylamino, C₁-C₃ alkylaminocarbonyl, C₁-C₃ alkoxycarbonyl, amino, hydroxy, nitro, azido, trifluoromethyl, cyano or halogen.

5. Tricyclic thiazolo[3,2-c]pyrimidine and thiazino[3,2-c]pyrimidine derivatives as claimed in claims 1-4, wherein R¹ denotes hydrogen, C₁-C₃ alkyl, C₂-C₄ alkenyl, C₂-C₄ alkinyl, C₁-C₃ alkoxy, C₁-C₃ alkylmercapto, C₁-C₃ alkylamino, C₁-C₃ alkoxycarbonyl, amino, halogen, hydroxy, nitro, cyano and azido.

6. Tricyclic thiazolo[3,2-c]pyrimidine and thiazino[3,2-c]pyrimidine derivatives as claimed in claims 1-5, wherein A denotes a phenyl or pyridinyl ring which can be substituted by a halogen atom, R²-R⁶ each represent a hydrogen atom, m and n represent the number 0 and X represents an oxygen or sulphur atom.

7. Process for the production of tricyclic thiazolo[3,2-c]pyrimidine and thiazino[3,2-c]pyrimidine derivatives as claimed in claims 1-6, wherein compounds of the general formula II 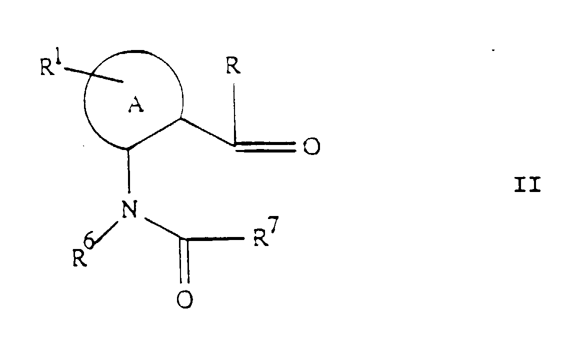 in which A, R, R¹ and R⁶ have the meaning stated above and R⁷ is a readily cleavable group such as CCl₃, CF₃, OR⁸ or NR⁸R⁹ in which R⁸ and R⁹ can be the same or different and can for example denote C₁-C₆ alkyl or substituted or unsubstituted phenyl, phenylalkyl, hetaryl or hetarylalkyl, are reacted in a suitable inert solvent at room temperature to reflux temperature possibly in the presence of catalytic amounts of an acid or a base with a substituted or unsubstituted cysteamine or a 3-mercapto-1-propanamine of the general formula III 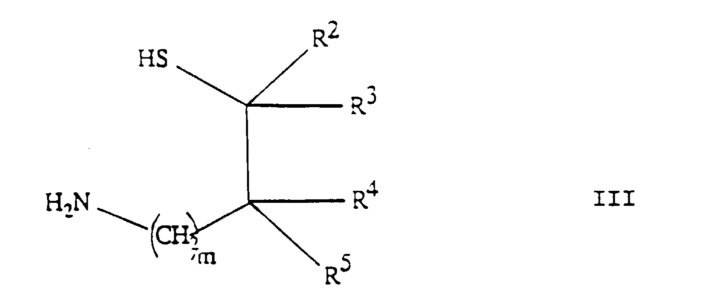 in which R², R³, R⁴, R⁵ and m have the stated meaning and subsequently if desired, compounds of formula I in which X=0 are converted into compounds of formula I in which X=S or X=NH.

8. Pharmaceutical agent containing at least one tricyclic thiazolo[3,2-c]pyrimidine or thiazino[3,2-c]pyrimidine derivative of formula I as claimed in claims 1-6 as well as pharmacologically compatible auxiliary or carrier substances.

9. Use of tricyclic thiazolo[3,2-c]pyrimidine or thiazino[3,2-c]pyrimidine derivatives as claimed in claims 1-6 for the production of pharmaceutical agents having antiviral action.

10. Process for the production of pharmaceutical agents as claimed in claim 7, wherein at least one compound of formula I is mixed with pharmacologically compatible auxiliary substances and processed into pharmaceutical forms of administration.

## Revendications

1. Dérivés tricycliques de thiazolo[3,2-c]pyrimidine et de thiazino[3,2-c]pyrimidine, de formule I 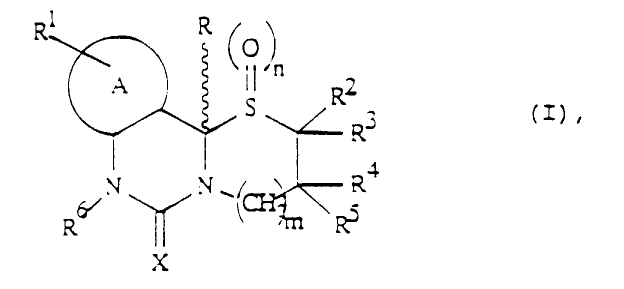 dans laquelle
A représente un noyau carbocyclique ayant 5 ou 6 atomes de carbone ou un noyau hétérocyclique ayant au maximum 4 hétéroatomes, les hétéroatomes pouvant être identiques ou différents et représentant un atome d'oxygène, d'azote ou de soufre, et un ou plusieurs atomes d'azote des hétérocycles pouvant porter éventuellement un atome d'oxygène, et A est éventuellement substitué par un ou plusieurs restes R¹ qui peuvent être identiques ou différents,
X représente un atome d'oxygène ou de soufre ou le groupe =NH, =N(alkyle en C₁-C₆) ou =S(O)₂,
R représente un reste aliphatique saturé ou insaturé, linéaire ou ramifié, ayant de 1 à 9 atomes de C, qui peut porter un substituant phényle, ou
un noyau phényle, ou
un noyau carbocyclique mono-, bi- ou tricyclique ayant de 7 à 15 atomes de C ou un système hétérocyclique mono-, bi- ou tricyclique, dont chaque cycle comporte 5 ou 6 atomes et chaque système cyclique peut comprendre 1-4 ou 1-5 hétéroatomes, les hétéroatomes étant l'azote, le soufre ou l'oxygène, et le noyau phényle, le noyau carbocyclique mono-, bi- ou tricyclique ou le système hétérocyclique mono-, bi- ou tricyclique porte éventuellement un ou plusieurs substituants tels qu'un groupe alkyle en C₁-C₆, alcoxy en C₁-C₆, (alkyle en C₁-C₆)mercapto, (alkyle en C₁-C₆)sulfinyle, (alkyle en C₁-C₆)sulfonyle, alcényle en C₂-C₆, alcinyle en C₂-C₆, alcényloxy en C₂-C₆, (alcényle en C₂-C₆)mercapto, alcinyloxy en C₂-C₆, (alcinyle en C₂-C₆)mercapto, amino, (alkyle en C₁-C₆)amino, di(alkyle en C₁-C₆)amino, (alkyle en C₁-C₆)carbonylamino, (alkyle en C₁-C₆)amino-carbonyle, (alcoxy en C₁-C₆)carbonyle, hydroxy, benzyloxy, phénylmercapto, phényloxy, nitro, cyano, halogéno, trifluorométhyle, azido, formylamino, carboxy ou phényle,
R¹ représente un atome d'hydrogène, un reste aliphatique saturé ou insaturé, linéaire ou ramifié, ayant de 1 à 6 atomes de C ou un groupe alcoxy en C₁-C₆, (alkyle en C₁-C₆)mercapto, (alkyle en C₁-C₆)sulfinyle, (alkyle en C₁-C₆)sulfonyle, amino, (alkyle en C₁-C₆)amino, di(alkyle en C₁-C₆)amino, sulfonamido, (alcoxy en C₁-C₆)carbonyle, carboxy, halogéno, hydroxy, nitro, cyano, azido, phényle ou benzyloxy,
R², R³, R⁴ et R⁵ peuvent être identiques ou différents et peuvent représenter chacun, indépendamment les uns des autres, un atome d'hydrogène, un groupe alkyle en C₁-C₆, hydroxy(alkyle en C₁-C₆), cyano, carboxy ou (alcoxy en C₁-C₆)carbonyle, ou R² et R⁴ peuvent représentent une autre liaison entre les atomes de C auxquels ils sont liés,
R⁶ représente un atome d'hydrogène ou un groupe alkyle en C₁-C₆,
n vaut 0, 1 ou 2 et
m vaut 0 ou 1,
ainsi que leurs tautomères, énantiomères, diastéréomères et leurs sels physiologiquement acceptables.

2. Dérivés tricycliques de thiazolo[3,2-c]pyrimidine et de thiazino[3,2-c]pyrimidine, selon la revendication 1, caractérisés par le fait que A représente un noyau phényle substitué une fois par R¹ ou un hétérocycle choisi dans le groupe constitué par un cycle furanyle, thiophényle, pyrrolyle, oxazolyle, isoxazolyle, thiazolyle, pyrazolyle, imidazolyle, oxadiazolyle, triazolyle, pyridinyle, pyridazinyle, pyrimidinyle ou pyrazinyle.

3. Dérivés tricycliques de thiazolo[3,2-c]pyrimidine et de thiazino[3,2-c]pyrimidine, selon la revendication 1 ou 2, caractérisés par le fait que R représente un groupe phényle ou un noyau carbocyclique choisi dans le groupe constitué par les restes naphtyle, anthracényle, phénanthrényle, fluorényle, indényle, indanyle, acénaphtényle, norbornyle, adamantyle, cycloalkyle en C₃-C₇ ou cycloalcényle en C₅-C₈, ces restes pouvant être non substitués ou substitués par un groupe alkyle en C₁-C₆, alcoxy en C₁-C₆, (alkyle en C₁-C₆)mercapto, (alkyle en C₁-C₆)sulfinyle, (alkyle en C₁-C₆)sulfonyle, alcényle en C₂-C₆, alcinyle en C₂-C₆, alcényloxy en C₃-C₆, (alkyle en C₁-C₆)amino, di(alkyle en C₁-C₆)amino, (alkyle en C₁-C₆)carbonyl-amino, (alkyle en C₁-C₆)amino-carbonyle, (alcoxy en C₁-C₆)carbonyle, amino, hydroxy, nitro, azido, trifluorométhyle, cyano ou halogéno.

4. Dérivés tricycliques de thiazolo[3,2-c]pyrimidine et de thiazino[3,2-c]pyrimidine, selon la revendication 1 ou 2, caractérisés par le fait que R représente un reste hétérocyclique choisi dans le groupe constitué par les systèmes pyridine, pyrimidine, pyridazine, pyrazine, triazine, pyrrol, pyrazole, imidazole, triazole, thiazole, oxazole, isoxasole, oxadiazole, furazane, furane, thiophène, indol, quinoléine, isoquinoléine, coumarone, thionaphtène, benzoxazole, benzothiazole, indazole, benzimidazole, benzotriazole, chromène, phtalazine, quinazoline, quinoxaline, méthylènedioxybenzène, carbazole, acridine, phénoxazine, phénothiazine, phénazine ou purine, ces hétérocycles pouvant être partiellement ou totalement hydrogénés et pouvant être non substitués ou substitués par un groupe alkyle en C₁-C₃, alcoxy en C₁-C₃, (alkyle en C₁-C₃)mercapto, (alkyle en C₁-C₃)sulfinyle, (alkyle en C₁-C₃)sulfonyle, alcényle en C₂-C₄, alcinyle en C₂-C₃, alcényloxy en C₃-C₄, (alkyle en C₁-C₃)amino, di(alkyle en C₁-C₃)amino, (alkyle en C₁-C₃)carbonyl-amine, (alkyle en C₁-C₃)amino-carbonyle, (alcoxy en C₁-C₃)carbonyle, amino, hydroxy, nitro, azido, trifluorométhyle, cyano ou halogéno.

5. Dérivés tricycliques de thiazolo[3,2-c]pyrimidine et de thiazino[3,2-c]pyrimidine, selon les revendications 1 à 4, caractérisés par le fait que R¹ représente un atome d'hydrogène, un groupe alkyle en C₁-C₃, alcényle en C₂-C₄, alcinyle en C₂-C₄, alcoxy en C₁-C₃, (alkyle en C₁-C₃)mercapto, alkylamino en C₁-C₃, (alcoxy en C₁-C₃)carbonyle, amino, halogéno, hydroxy, nitro, cyano et azido.

6. Dérivés tricycliques de thiazolo[3,2-c]pyrimidine et de thiazino[3,2-c]pyrimidine, selon les revendications 1 à 5, caractérisés par le fait que A représente un noyau phényle ou pyridinyle qui peut être substitué par un atome d'halogène, R²-R⁶ représentent chacun un atome d'hydrogène, m et n valent 0 et X représente un atome d'oxygène ou de soufre.

7. Procédé de préparation de dérivés tricycliques de thiazolo[3,2-c]pyrimidine et de thiazino[3,2-c]pyrimidine, selon les revendications 1 à 6, caractérisés par le fait que l'on fait réagir des composés de formule générale Il 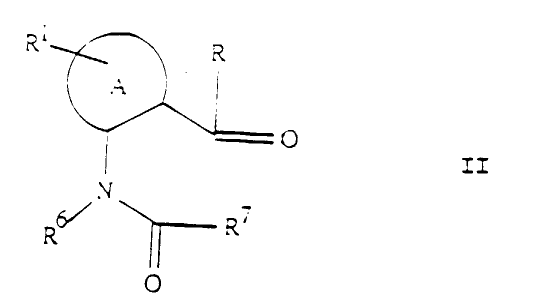 dans laquelle A, R, R¹ et R⁶ ont les significations indiquées ci-dessus, et R⁷ est un groupe facilement séparable tel que CCl₃, CF₃, OR⁸ ou NR⁸R⁹, R⁸ et R⁹ pouvant être identiques ou différents et pouvant représenter par exemple un groupe alkyle en C₁-C₆ ou un groupe phényle, phénylalkyle, hétéroaryle ou hétéroalkyle substitué ou non substitué, avec une cystéamine ou 3-mercapto-1-propanamine substituée ou non substituée, de formule générale III 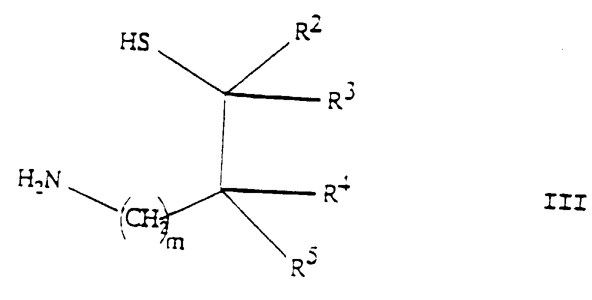 dans laquelle R², R³, R⁴, R⁵ et m ont les significations indiquées ci-dessus, dans un solvant inerte approprié, à une température comprise entre la température ambiante et la température de reflux, éventuellement en présence d'une quantité catalytique d'acide ou de base et ensuite, on transforme éventuellement ultérieurement les composés de formule I où X=O en composés de formule I où X=S ou X=NH.

8. Médicament contenant au moins un dérivé tricyclique de thiazolo[3,2-c]pyrimidine et de thiazino[3,2-c]pyrimidine de formule I selon les revendications 1-6, ainsi que des adjuvants et véhicules pharmacologiquement acceptables.

9. Utilisation de dérivés tricycliques de thiazolo[3,2-c]pyrimidine et de thiazino[3,2-c]pyrimidine selon les revendications 1-6, pour la préparation de médicaments ayant une activité antivirale.

10. Procédé de préparation de médicaments selon la revendication 7, caractérisé par le fait qu'on mélange au moins un composé de formule I avec des adjuvants pharmaceutiquement acceptables et on les met sous une forme galénique.
